# EUROPÄISCHE PATENTSCHRIFT

(11) **EP 2 480 460 B1**
(45) Veröffentlichungstag und Bekanntmachung des Hinweises auf die Patenterteilung: **04.06.2014**
(21) Anmeldenummer: 10787265.7
(22) Anmeldetag: 17.09.2010
(51) Int. Cl.: B65B 57/00, G01N 21/35, G01N 21/95, G01N 33/15

(54) **VORRICHTUNG UND VERFAHREN ZUR KONTROLLE DES INHALTS MEDIKAMENTE ENTHALTENDER PACKUNGSEINHEITEN**
DEVICE AND METHOD FOR INSPECTING THE CONTENT OF PACKAGING UNITS CONTAINING MEDICATIONS
DISPOSITIF ET PROCÉDÉ DE CONTRÔLE DU CONTENU D'UNITÉS D'EMBALLAGE CONTENANT DES MÉDICAMENTS

(30) Priorität: 23.09.2009 DE 202009012784 U; 30.03.2010 DE 102010013335
(43) Veröffentlichungstag der Anmeldung: 01.08.2012
(73) Patentinhaber: Steinweg, Detlef, 44575 Castrop-Rauxel (DE)
(72) Erfinder: Steinweg, Detlef, 44575 Castrop-Rauxel (DE)
(74) Vertreter: Bernhardt, Reinhold
(86) Internationale Anmeldenummer: PCT/DE2010/001130
(87) Internationale Veröffentlichungsnummer: WO 2011/035775

(56) Entgegenhaltungen:
- US-A1- 2004 207 842
- US-A1- 2007 008 523
- US-A1- 2007 263 212
- US-B1- 6 357 495
- US-B1- 6 647 702

## Beschreibung

Die Erfindung betrifft eine Vorrichtung zur Kontrolle des Inhalts von Packungseinheiten, die wenigstens ein Exemplar einer Darreichungseinheit eines Arzneimittels enthalten, mit einer Sensoreinrichtung zum Empfang und zur Verarbeitung von dem Inhalt einer Packungseinheit ausgehenden, durch eine transparente Wand der Packungseinheit tretenden Lichts, sowie mit einer mit der Sensoreinrichtung verbundenen, dem Inhalt der Packungseinheit einen für diesen vorgeschriebenen Inhalt gegenüberstellenden Vergleichseinrichtung, und wobei die Sensoreinrichtung Einrichtung zur Ermittlung eines zu dem Inhalt der Packungseinheit charakteristischen Reflexions- oder Absorptionsspektrums SG umfasst. Die Erfindung betrifft ferner ein mit Hilfe der Vorrichtung durchführbares Verfahren gemäß Oberbegriff des Anspruchs 11.

Eine solche Vorrichtung und ein solches Verfahren dienen insbesondere der Kontrolle des Inhalts von Packungseinheiten, die z.B. individuell für Patienten zusammengestellte Arzneimittelgaben enthalten.

Die Sensoreinrichtung durch Benutzung bekannter derartiger Kontrollvorrichtungen umfasst eine den Packungsinhalt bildlich erfassende Kamera sowie Einrichtungen zur Bildverarbeitung, welche z.B. Tabletten oder Kapseln anhand ihrer Form, Abmessungen, ihres Grauwerts oder/und ihrer Farbe identifizieren und die Anzahl von Tabletten oder/und Kapseln eines Medikaments ermitteln. Sind die Tabletten oder Kapseln verschiedener Medikamente äußerlich ähnlich, so versagt die Identifizierung.

Ein Verfahren und eine Vorrichtung der eingangs genannten Art geht aus der US 2004/207842 A1 hervor. Ein in einem Arzneifläschen vorhandenes Medikament wird anhand eines Vergleichsspektrums und eines gemessenen Reflexionsspektrums identifiziert, so dass ein Arzneifläschen tatsächlich das Medikament enthält, welches einem Patienten, für welchen das Fläschen bestimmt ist, verschrieben wurde.

Die US 2007/263212 A1 beschreibt ein Verfahren und eine Vorrichtung, welche anorganisches Schüttgut in einem Schüttgutstrom anhand gemessener Reflexisonsspektren und Vergleichsspektren analysiert.

Der Erfindung liegt die Aufgabe zugrunde, eine neue Vorrichtung und eine neues Verfahren der eingangs erwähnten Art zu schaffen, die bzw. das eine zuverlässige Kontrolle unterschiedliche Medikamente umfassende Arzneimittelzusammenstellungen für Patienten ermöglicht.

Diese Aufgabe wird durch die in den unabhängigen Ansprüchen 1 und 11 angegebenen Merkmale gelöst.

Indem das Reflexions- oder Absorptionsspektrum in erster Linie von der stofflichen Zusammensetzung und weniger von der äußeren Erscheinung eines Medikaments abhängt, gelingt zuverlässig dessen Identifizierung.

Als zur Unterscheidung und Identifizierung besonders geeignet haben sich Reflexions- oder Absorptionsspektren im Infrarotbereich erwiesen. Insbesondere ist die genannte Einrichtung zur Ermittlung eines Spektrums im nahen Infrarotbereich vorgesehen.

Vorzugsweise umfasst die Sensoreinrichtung zusätzlich Einrichtungen zur bildlichen Erfassung des Inhalts der Packungseinheiten, und es sind ferner Einrichtungen zur Bildverarbeitung vorhanden. Durch diese zusätzlichen Einrichtungen lässt sich neben einer Identifizierung der Arzneimittel als solche feststellen, wie viele Darreichungseinheiten, z.B: Tabletten oder Kapseln, eines Arzneimittels in der Packungseinheit enthalten sind.

Bei den Packungseinheiten handelt es sich z.B. um allseitig geschlossene transparente Schlauchbeutel. Als weitere bevorzugte Ausführungsform kommen Blisterverpackungen in Betracht.

Die Packungseinheiten können der Sensoreinrichtung in einem zusammenhängenden, vorzugsweise von einer Rolle abwickelbaren oder/und auf eine Rolle aufwickelbaren Strang zugeführt werden.

Zweckmäßig ist eine Transporteinrichtung vorhanden, die die Packungseinheiten der Sensoreinrichtung getaktet zuführt.

In weiterer Ausgestaltung der Erfindung umfasst die Vorrichtung eine Einrichtung zur Erfassung einer Markierung auf den Packungseinheiten, insbesondere einen Barcode-Leser. So können die einzelnen Packungseinheiten identifiziert und in Zuordnung zur ermittelten Identifizierungsinformation kontrolliert werden. Alternativ könnte die Zuordnung auch nur aufgrund der Reihenfolge der Packungseinheiten oder/und einer von einer übergeordneten EDV-Einrichtung eingegebenen Identifizierungsinformation erfolgen.

Zweckmäßig weist die Kontrollvorrichtung ferner eine Einrichtung zur Markierung oder/und Aussonderung als fehlerhaft erkannter Packungseinheiten auf.

Vorzugsweise sind die ermittelten Reflexionsspektren und Bilder sowie Untersuchungsergebnisse speicherbar, insbesondere diejenigen von als fehlerhaft erkannten Packungseinheiten.
In weiterer Ausgestaltung der Erfindung kann in die Kontrollvorrichtung eine die Packungseinheiten herstellende Verpackungsvorrichtung oder umgekehrt integriert sein.

Gemäß dem erfindungsgemäßen Verfahren wird unter Detektierung von dem Inhalt einer Packungseinheit ausgehenden Lichts kontrolliert, ob der vorhandene Inhalt seinem vorgeschriebenen Inhalt entspricht, wobei ein dem vorhandenen Inhalt entsprechendes Reflexions- oder Absorptionsspektrum ermittelt und mit einem dem vorgeschriebenen Inhalt entsprechenden solchen Spektrum verglichen wird.

Solche Vergleichsspektren könnten für alle in Frage kommenden Packungsinhalte gespeichert sein. Gemäß Erfindung sind jedoch anstelle derartiger Gesamtspektren nur die Einzelspektren für alle in Frage kommenden unterschiedlichen Darreichungseinheiten gespeichert.

Zum indirekten Vergleich des ermittelten Spektrums mit dem Spektrum, das dem vorgeschriebenen Inhalt entspricht, wird das ermittelte Spektrum durch eine Linearkombination der gespeicherten Einzelspektren approximiert, wobei die Approximation unter Minimierung des mit einem Exponenten versehenen Residuums erfolgt und die Koeffizienten der Linearkombination sowie das minimierte Residuum jeweils mit vorgegebenen Schwellenwerten verglichen werden.

In einer besonders bevorzugten Ausführungsform der Erfindung wird nicht das quadratische Residuum minimiert, sondern ein Exponent > 2 gewählt, vorzugsweise der Exponent 4, 6 oder 8. Strukturunterschiede zwischen ähnlichen Spektren treten auf diese Weise deutlicher hervor.

Zur Eliminierung der Schwankungen von Vorrichtungsparametern werden vorzugsweise normierte Spektren verwendet. Insbesondere erfolgt eine Normierung der Lichtintensität auf einen Mittelwert 0 und einen Wert 1 der Standardabweichung vom Mittelwert.

Die Erfindung wird nachfolgend anhand eines Ausführungsbeispiels und der beiliegenden, sich auf dieses Ausführungsbeispiel beziehenden Figur weiter erläutert.

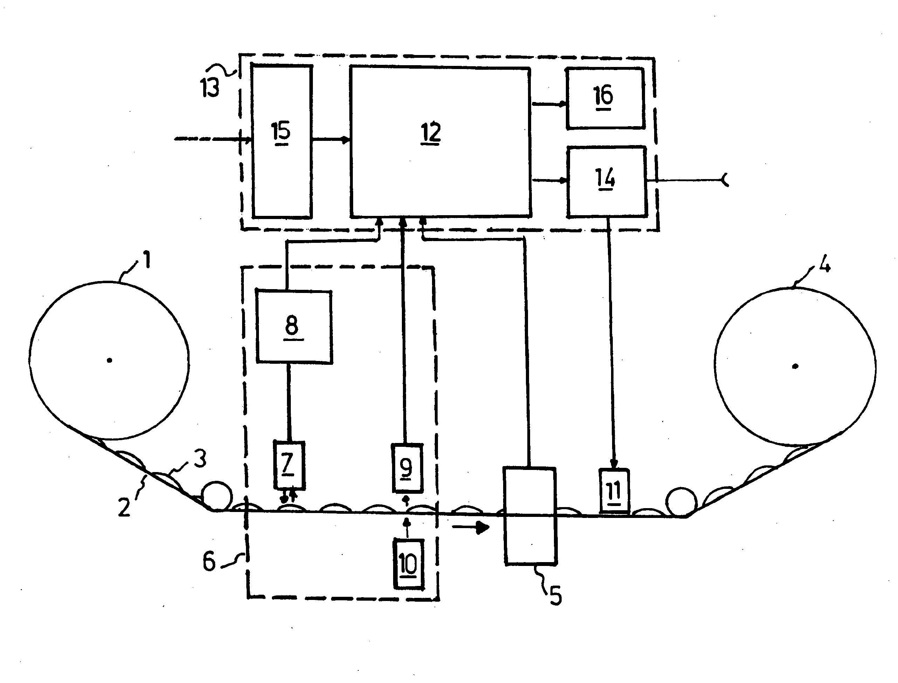

Eine in der Figur schematisch darstellte Vorrichtung zur Kontrolle des Inhalts vorgeschriebene Medikamentzusammenstellungen enthaltender Packungseinheiten umfasst eine Zuführungsrolle 1, auf welcher ein Strangs 2 zusammenhängender, verschlossene Packungseinheiten bildender Schlauchbeutel 3 aufgewickelt ist. Die transparenten Schlauchbeutel enthalten z.B. individuell für Patienten zusammengestellte Arzneimittelfüllungen in Form von Tabletten, Kapseln oder/und anderer Darreichungseinheiten.

Der von der Rolle 1 abgewickelte, durch die Vorrichtung gelaufene Strang 2 lässt sich auf eine Rolle 4 wieder aufwickeln. Eine den Strang 2 bewegende Transporteinrichtung 5 sorgt für einen getakteten Durchlauf des Strangs 2 durch die Vorrichtung.

Die Schlauchbeutel 3 erreichen zuerst eine Sensoreinrichtung 6. Die Sensoreinrichtung 6 umfasst einen Sende-/Empfängerkopf 7, welcher Licht im nahen Infrarotbereich aussendet und reflektiertes Licht empfängt. Der Sende-/ Empfängerkopf 7 steht in Verbindung mit einer das empfangene Licht analysierenden Spektrometereinrichtung 8.

Die Sensoreinrichtung 6 umfasst ferner einen dem Sende-/Empfängerkopf 7 in Bewegungsrichtung des Strangs 2 nachgeordnete digitale Kamera 9 und eine der Kamera 9 gegenüberliegende Durchlicht-Beleuchtungseinrichtung 10. Alternativ oder zusätzlich könnte eine Auflicht-Beleuchtungseinrichtung vorhanden sein.

Vor der Aufwicklung auf die Rolle 4 passiert der Strang 2 eine Markierungseinrichtung 11.

Die genannte Spektrometereinrichtung 8, die digitale Kamera 9 und die Transporteinrichtung 5 stehen in Verbindung mit einer Steuer- und Verarbeitungseinrichtung 12, die durch einen industriellen Personalcomputer 13 gebildet ist. Der Personalcomputer 13 umfasst ferner eine Ausgabeeinrichtung 14, über welche die Markierungseinrichtung 11 ansteuerbar ist, sowie eine Eingabeeinrichtung 15 und eine Anzeigeeinrichtung 16.

Im Kontrollbetrieb werden die zu dem Strang 2 verbundenen Schlauchbeutel 3 mit Hilfe der Transporteinrichtung 5 taktweise vorgeschoben. Jeder der Schlauchbeutel 3 kann einen Barcode tragen, durch den er über einen (nicht gezeigten) mit der Eingabeeinrichtung 15 verbundenen Barcode-Leser identifizierbar ist. Alternativ oder zusätzlich kann die Identifikation durch Eingabe entsprechender Informationen aus einem übergeordneten EDV-System, z.B. ERP-System, erfolgen. Schließlich ist auch eine Identifizierung lediglich über die Reihenfolge der Schlauchbeutel 3 möglich.

Nach der Identifizierung kann der für den betreffenden Schlauchbeutel vorgeschriebene Inhalt aus einer Datei, z.B. einer Datei einer zentralen Datenbank, abgerufen werden. Insbesondere werden Sollwerte abgerufen, die für die vorgeschriebenen Medikamente und deren Mengen bzw. Toblettenanzahlen repräsentativ sind.

Die Spektrometereinrichtung 8 ermittelt anhand der von dem Sende-/Empfängerkopf 7 übertragenen Signale für jeden Schlauchbeutel 3 ein Reflexionsspektrum, dass für die stoffliche Zusammensetzung des in dem betreffenden Schlauchbeutel 3 enthaltenen Medikaments charakteristisch ist. Sind mehrere unterschiedliche Medikamente darin enthalten, so wird ein in Einzelspektren entsprechender Anzahl zerlegbares Gesamtspektrum ermittelt.

Die Steuer- und Verarbeitungseinrichtung 12 vergleicht die ermittelten Spektren mit den abgerufenen Sollwerten bzw. Sollwertspektren und identifiziert so die betreffenden Medikamente.

Die Steuer- und Verarbeitungseinrichtung 12 analysiert ferner die von der digitalen Kamera 9 aufgenommenen Bilder und ermittelt durch Verarbeitung von Form, Abmessungen, Grauwerten oder/und Farben die Anzahl der ein identifiziertes Medikament betreffenden Tabletten oder/und Kapseln. Bei der Zuordnung der Anzahlen zu den Medikamenten können für die Menge der Medikamente repräsentative Intensitätswerte des gemessenen Reflexionslichts herangezogen werden.

Werden die abgerufenen Sollwerte durch die ermittelten Werte nicht bestätigt, so erfolgt eine Markierung des betreffenden Schlauchbeutels mit Hilfe der Markierungseinrichtung 11.

Zu Dokumentationszwecken wird in Zuordnung zur Identifizierungsinformation jedes Beutels 3 ein Bild zusammen mit dem aufgenommenen Reflexionsspektrum sowie dem Ergebnis der Kontrolle in einer Datenbank abgelegt.
Die abgerufenen Sollwerte können bei Bedarf durch die Anzeigeeinrichtung 16 dargestellt werden.

Es versteht sich, dass bei nicht zusammenhängenden Packungseinheiten unmittelbar nach Erkennung einer fehlerhaften Befüllung eine Aussonderung der betreffenden Packungseinheit erfolgen kann.

In der oben genannten Datei sind Reflexions- oder Absorptionsspektren für alle in Frage kommenden Medikamente bzw. Darreichungseinheiten gespeichert. Umfasst der für eine Packungseinheit vorgeschriebene Inhalt mehrere Medikamente und damit mehrere, zumindest durch das enthaltene Medikament unterscheidbare Darreichungseinheiten, so werden die betreffenden Spektren S 1 bis SN abgerufen.

Bei den Spektren S1 bis SN handelt es sich um normierte Spektren, die vorzugsweise auf einen Mittelwert 0 und eine Standardabweichung 1 vom Mittelwert bezogen sind. Das für die betreffende Packungseinheit ermittelte Gesamtspektrum SG wird in gleicher Weise normiert.

Ein Vergleich des für eine Packungseinheit ermittelten Gesamtspektrums SG mit dem Gesamtspektrum, das dem vorgeschriebenen Inhalt dieser Packungseinheit entspricht, erfolgt indirekt dadurch, dass das ermittelte Spektrum SG durch eine Linearkombination der Spektren S1 bis SN mit Koeffizienten A1 bis AN approximiert wird. Die Approximation erfolgt vorzugsweise unter Minimierung des Residuums mit einem Exponenten P > 2, z.B. P = 4, 6 oder 8. Durch den Exponenten P > 2 lassen sich strukturelle Unterschiede zwischen einander ähnlichen Spektren besser kenntlich machen.

Ergibt die Approximation, dass alle Koeffizienten A1 bis AN über einem für jeden dieser Koeffizienten vorgegebenen Schwellenwert liegen und dass das Residuum kleiner als ein dafür vorgegebener Schwellenwert ist, so stimmt der Inhalt der betreffenden Packungseinheit wenigstens in Bezug auf die Anzahl darin enthaltener unterschiedlichen Arten von Darreichungseinheiten mit dem für diese Packungseinheit vorgeschriebenen Inhalt überein. Liegt zumindest einer der Koeffizienten unter seinem Schwellenwert, so fehlt in der Packungseinheit das betreffende Medikament. Liegt das Residuum über dem betreffenden Schwellenwert, so enthält die Packungseinheit zumindest ein dafür nicht vorgeschriebenes Medikament.

## Patentansprüche

1. Vorrichtung zur Kontrolle des Inhalts von Packungseinheiten (3), die wenigstens ein Exemplar einer Darreichungseinheit eines Arzneimittels enthalten, mit einer Sensoreinrichtung (6) zum Empfang und zur Verarbeitung von dem Inhalt einer Packungseinheit (3) ausgehendes, durch eine transparente Wand der Packungseinheit (3) tretendes Lichts empfangt und verarbeitet, sowie mit einer mit der Sensoreinrichtung (6) verbundenen, dem Inhalt der Packungseinheit (3) einen für diese vorgeschriebenen Inhalt gegenüberstellenden Vergleichseinrichtung (12), wobei die Sensoreinnchtung (6) Einnchtungen (7,8) zur Ermittlung eines für den Inhalt der Packungseinheit (3) charakteristischen Reflexions- oder Absorptionsspektrums SG umfasst,
**dadurch gekennzeichnet,**
**dass** die Vergleichseinrichtung (12) zur Speicherung von Reflexions- oder Absorptionsspektren S1 bis SN für N verschiedene Arten vorgeschriebener Darreichungseinheiten, zur Approximation des ermittelten Spektrums SG durch eine Linearkombination der Spektren S1 bis SN unter Minimierung des mit einem Exponenten p versehenen Residuums und zum Vergleich der Koeffizienten A1 bis AN der Linearkombination sowie des minimierten Residuums mit vorgegebenen Schwellenwerten vorgesehen ist

2. Vorrichtung nach Anspruch 1,
**dadurch gekennzeichnet,**
**dass** die genannte Einrichtungen (7,8) zur Ermittlung eines Reflexions- oder Absorptionsspektrums im Infrarotbereich, vorzugsweise nahen Infrarotbereich, vorgesehen sind

3. Vorrichtung nach Anspruch 1 oder 2,
**dadurch gekennzeichnet,**
**dass** die Sensoreinrichtung (6) ferner Einrichtungen (9,10) zur bildlichen Erfassung des Inhalts der Packungseinheit (3) umfasst.

4. Vorrichtung nach Anspruch 3,
**dadurch gekennzeichnet,**
**dass** Einrichtungen (12) zur Bildverarbeitung vorgesehen sind.

5. Vorrichtung nach einem der Ansprüche 1 bis 4,
**dadurch gekennzeichnet,**
**dass** die Packungseinheiten allseitig geschlossene Schlauchbeutel (3) oder Blisterverpackungen sind

6. Vorrichtung nach einem der Ansprüche 1 bis 5,
**dadurch gekennzeichnet,**
**dass** die Packungseinheiten (3) der Sensoreinrichtung (6) in einem zusammenhängenden, vorzugsweise von einer Rolle (1) abwickelbaren oder/und auf eine Rolle (4) aufwickelbaren, Strang (2) zuführbar sind.

7. Vorrichtung nach einem der Ansprüche 1 bis 6,
**dadurch gekennzeichnet,**
**dass** die Packungseinheiten (3) der Sensoreinrichtung (6) durch eine Transporteinrichtung (5) getaktet zuführbar sind.

8. Vorrichtung nach einem der Ansprüche 1 bis 7,
**dadurch gekennzeichnet,**
**dass** eine Einrichtung zur Erkennung einer Markierung auf den Packungseinheiten (3), insbesondere eines Barcodes, vorgesehen ist.

9. Vorrichtung nach einem der Ansprüche 1 bis 8,
**dadurch gekennzeichnet,**
**dass** eine Einrichtung (11) zur Markierung oder/und Aussonderung als fehlerhaft erkannter Packungseinheiten (3) vorgesehen ist.

10. Vorrichtung nach einem der Ansprüche 1 bis 9,
**dadurch gekennzeichnet,**
**dass** in die Kontrollvorrichtung eine Verpackungsvorrichtung zur Herstellung der Packungseinheiten (3) integriert ist.

11. Verfahren zur Kontrolle des Inhalts von Packungseinheiten (3), die jeweils wenigstens ein Exemplar einer Darreichungseinheit eines Arzneimittels enthalten, wobei unter Detektierung von dem Arzneimittel ausgehenden Lichts kontrolliert wird, ob der vorhandene Inhalt einer Packungseinheit (3) einem für diese Packungseinheit (3) vorgeschriebenen Inhalt entspricht, und ein dem Inhalt der Packungseinheit (3) entsprechendes Reflexions- oder
Absorptionsspektrum SG ermittelt und mit einem dem vorgeschriebenen Inhalt entsprechenden solchen Spektrum verglichen wird,
**dadurch gekennzeichnet,**
**dass** Reflexions- oder Absorptionsspektren S1 bis SN für N verschiedene Arten vorgeschriebener Darreichungseinheiten gespeichert werden, und dass das ermittelte Spektrum SG durch eine Linearkombination der Spektren S1 bis SN unter Minimierung des mit einem Exponenten p versehenen Residuums approximiert wird und die Koeffizienten A1 bis AN der Linearkombination sowie das minimierte Residuum mit vorgegebenen Schwellenwerten verglichen werden.

12. Verfahren nach Anspruch 11,
**dadurch gekennzeichnet,**
**dass** der Exponent p > 2 gewählt wird und vorzugsweise 4, 6 oder 8 beträgt.

13. Verfahren nach einem der Ansprüche 11 oder 12,
**dadurch gekennzeichnet,**
**dass** die Spektren SG sowie S1 bis SN normiert werden und vorzugsweise eine Normierung der Lichtintensität auf einen Mittelwert 0 und eine Standardabweichung 1 vom Mittelwert erfolgt.

## Claims

1. Device for inspecting the content of packaging units (3), which contain at least one specimen of a dosage unit of a medicament, comprising a sensor apparatus (6) for receiving and processing light emanating from the content of a packaging unit (3) and passing through a transparent wall of the packaging unit (3), and comprising a comparison apparatus (12), which is connected to the sensor apparatus (6) and compares the content of the packaging unit (3) with a prescribed content therefor, wherein the sensor apparatus (6) comprises apparatuses (7, 8) for establishing a reflection or absorption spectrum SG characteristic to the content of the packaging unit (3),
**characterized**
**in that** the comparison apparatus (12) is provided for storing reflection or absorption spectra S1 to SN for N different types of prescribed dosage units, for approximating the established spectrum SG by a linear combination of spectra S1 to SN while minimizing the residual provided with an exponent p, and for comparing the coefficients A1 to AN of the linear combination and of the minimized residual with predetermined thresholds.

2. Device according to Claim 1,
**characterized**
**in that** the aforementioned apparatuses (7, 8) are provided for establishing a reflection or absorption spectrum in the infrared range, preferably in the near-infrared range.

3. Device according to Claim 1 or 2,
**characterized**
**in that** the sensor apparatus (6) furthermore comprises apparatuses (9, 10) for image acquisition of the content of the packaging unit (3).

4. Device according to Claim 3,
**characterized**
**in that** apparatuses (12) for image processing are provided.

5. Device according to one of Claims 1 to 4,
**characterized**
**in that** the packaging units are pouches (3) or blister packs which are closed on all sides.

6. Device according to one of Claims 1 to 5,
**characterized**
**in that** the packaging units (3) can be fed to the sensor apparatus (6) in a continuous strand (2), which can preferably be unwound from a roller (1) and/or wound onto a roller (4).

7. Device according to one of Claims 1 to 6,
**characterized**
**in that** the packaging units (3) can be fed to the sensor apparatus (6) in a clocked manner by a transport apparatus (5).

8. Device according to one of Claims 1 to 7,
**characterized**
**in that** an apparatus for identifying a marking, in particular a barcode, on the packaging units (3) is provided.

9. Device according to one of Claims 1 to 8,
**characterized**
**in that** an apparatus (11) for marking and/or discarding packaging units (3) identified as faulty is provided.

10. Device according to one of Claims 1 to 9,
**characterized**
**in that** a packaging device for producing the packaging units (3) is integrated in the inspection device.

11. Method for inspecting the content of packaging units (3) which each contain at least one specimen of a dosage unit of a medicament, wherein, when detecting light emanating from the medicament, an inspection is carried out as to whether the present content of a packaging unit (3) corresponds to a content prescribed for this packaging unit (3), and a reflection or absorption spectrum SG corresponding to the content of the packaging unit (3) is established and compared to such a spectrum corresponding to the prescribed content,
**characterized**
**in that** reflection or absorption spectra S1 to SN for N different types of prescribed dosage units are stored and in that the established spectrum SG is approximated by a linear combination of the spectra S1 to SN while minimizing the residual provided with an exponent p, and the coefficients A1 to AN of the linear combination and the minimized residual are compared to predetermined thresholds.

12. Method according to Claim 11,
**characterized**
**in that** the exponent is selected as p > 2 and preferably equal to 4, 6 or 8.

13. Method according to either of Claims 11 and 12,
**characterized**
**in that** the spectra SG and S1 to SN are normalized and the light intensity is preferably normalized to a mean value of 0 and a standard deviation of 1 from the mean value.

## Revendications

1. Dispositif pour le contrôle du contenu d'unités d'emballage (3) qui contiennent au moins un exemplaire d'une unité d'administration d'un médicament, présentant un dispositif à capteur (6) pour recevoir et traiter une lumière partant du contenu d'une unité d'emballage (3), traversant une paroi transparente de l'unité d'emballage (3), ainsi qu'un dispositif de comparaison (12), relié au dispositif à capteur (6), confrontant le contenu de l'unité d'emballage (3) à un contenu prescrit pour celui-ci, le dispositif à capteur (6) comprenant des dispositifs (7,8) pour déterminer un spectre de réflexion ou d'absorption SG caractéristique pour le contenu de l'unité d'emballage (3), **caractérisé en ce que** le dispositif de comparaison (12) est destiné à mémoriser des spectres de réflexion ou d'absorption S1 à SN pour N types différents d'unités d'administration prescrites, à approximer le spectre déterminé SG par une combinaison linéaire des spectres S1 à SN en minimisant le résidu présentant un exposant p, à la puissance p, et à comparer les coefficients A1 à AN de la combinaison linéaire ainsi que le résidu minimisé à des valeurs seuil prédéfinies.

2. Dispositif selon la revendication 1, **caractérisé en ce que** les dispositifs mentionnés (7,8) sont destinés à déterminer un spectre de réflexion ou d'absorption dans la plage des infrarouges, de préférence des infrarouges proches.

3. Dispositif selon la revendication 1 ou 2, **caractérisé en ce que** le dispositif à capteur (6) comprend en outre des dispositifs (9,10) pour la détection par imagerie du contenu de l'unité d'emballage (3).

4. Dispositif selon la revendication 3, **caractérisé en ce que** les dispositifs (12) sont destinés au traitement d'images.

5. Dispositif selon l'une quelconque des revendications 1 à 4, **caractérisé en ce que** les unités d'emballage sont des sachets tubulaires (3) fermés de tous côtés ou des emballages blister.

6. Dispositif selon l'une quelconque des revendications 1 à 5, **caractérisé en ce que** les unités d'emballage (3) peuvent être acheminées vers le dispositif à capteur (6) sous forme d'un brin (2) d'un seul tenant, pouvant de préférence être déroulé d'un rouleau (1) et/ou enroulé sur un rouleau (4).

7. Dispositif selon l'une quelconque des revendications 1 à 6, **caractérisé en ce que** les unités d'emballage (3) peuvent être acheminées vers le dispositif à capteur (6) de manière synchronisée par un dispositif de transport (5).

8. Dispositif selon l'une quelconque des revendications 1 à 7, **caractérisé en ce qu'**il comporte un dispositif pour identifier un marquage sur les unités d'emballage (3), en particulier un barre-code.

9. Dispositif selon l'une quelconque des revendications 1 à 8, **caractérisé en ce qu'**il présente un dispositif (11) pour marquer et/ou pour retirer des unités d'emballage (3) identifiées comme défectueuses.

10. Dispositif selon l'une quelconque des revendications 1 à 9, **caractérisé en ce qu'**un dispositif d'emballage pour la fabrication des unités d'emballage (3) est intégré dans le dispositif de contrôle.

11. Procédé pour le contrôle du contenu d'unités d'emballage (3), qui contiennent à chaque fois au moins un exemplaire d'une unité d'administration d'un médicament, où on contrôle, par détection de la lumière partant du médicament, si le contenu présent d'une unité d'emballage (3) correspond à un contenu prescrit pour cette unité d'emballage (3) et on détermine un spectre de réflexion ou d'absorption SG correspondant au contenu de l'unité d'emballage (3) et on le compare avec un tel spectre correspondant au contenu prescrit, **caractérisé en ce que** des spectres de réflexion ou d'absorption S1 à SN pour N types différents d'unités d'administration prescrites sont mémorisées et **en ce que** le spectre déterminé SG est approximé par une combinaison linéaire des spectres S1 à SN en minimisant le résidu présentant un exposant p, et les coefficients A1 à AN de la combinaison linéaire ainsi que le résidu minimisé sont comparés à des valeurs seuil prédéfinies.

12. Procédé selon la revendication 11, **caractérisé en ce que** l'exposant p est choisi > 2 et vaut de préférence 4, 6 ou 8.

13. Procédé selon l'une quelconque des revendications 11 ou 12, **caractérisé en ce que** les spectres SG ainsi que S1 à SN sont normalisés et on réalise de préférence une normalisation de l'intensité lumineuse à une valeur moyenne de 0 et un écart-type de 1 de la valeur moyenne.
